(19) Europäisches Patentamt
European Patent Office
Office européen des brevets

(11) EP 0 635 496 B1

(12) # FASCICULE DE BREVET EUROPEEN

(45) Date de publication et mention de la délivrance du brevet: **29.04.1998 Bulletin 1998/18**

(51) Int. Cl.$^6$: **C07D 235/02**, A61K 31/415

(21) Numéro de dépôt: **94401548.6**

(22) Date de dépôt: **06.07.1994**

(54) **Nouveaux dérivés de benzospiroalcène, leur procédé de préparation et les compositions pharmaceutiques qui les contiennent**

Benzospiroalken-Derivate, Verfahren zu ihrer Herstellung und diese enthaltende pharmazeutische Zusammensetzungen

Benzospiroalkene derivatives, process for their preparation and pharmaceutical compositions containing them

(84) Etats contractants désignés:
**AT BE CH DE DK ES FR GB GR IE IT LI LU NL PT SE**

(30) Priorité: **20.07.1993 FR 9308860**

(43) Date de publication de la demande: **25.01.1995 Bulletin 1995/04**

(73) Titulaire: **ADIR ET COMPAGNIE**
**92415 Courbevoie Cédex (FR)**

(72) Inventeurs:
- **Cordi, Alex**
  **F-92150 Suresnes (FR)**
- **Lacoste, Jean-Michel**
  **F-92310 Sevres (FR)**
- **Laubie, Michel**
  **F-92420 Vaucresson (FR)**
- **Verbeuren, Tony**
  **F-78540 Vernouillet (FR)**
- **Descombes, Jean-Jacques**
  **F-93360 Neuilly-Plaisance (FR)**

(56) Documents cités:
- **JOURNAL OF MEDICINAL CHEMISTRY., vol.21, no.6, 1978, WASHINGTON US pages 585 - 587 P. A. CROOKS ET AL 'Synthesis of spiro(tetralin-2,2'-pyrrolidine) and spiro(indan-2,2'-pyrrolidine) derivatives as potential analgesics'**
- **JOURNAL OF THE CHEMICAL SOCIETY, PERKIN TRANSACTIONS 1., no.11, 1979, LETCHWORTH GB pages 2719 - 2726 P. A. CROOKS ET AL 'Synthesis of 5-hydroxy and 5,6-dihydroxy-derivatives of spiro(indane-2,2'-pyrrolidine), rigid analogues of tyramine and dopamine respectively'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.1/3.4

## Description

J. Med. Chem., 21, 585-587, 1978 et J. Chem. Soc, Perkin Transactions 1, 2719-2726, 1979 décrivent des composés spiro[(pyrrolidine)-2:2'(1',2',3',4'-tétrahydronaphtalène)] et spiro[(pyrrolidine)-2:2'(indane)] qui ont une activité analgésique.

La présente invention concerne des benzospiroalcènes, leur procédé de préparation et les compositions pharmaceutiques qui les contiennent, ainsi que leur utilisation en tant qu'agonistes $\alpha_1$ adrénergiques.
Le système nerveux adrénergique joue un rôle important à plusieurs niveaux, par exemple artériel, veineux, cardiaque, rénal et au niveau du système nerveux autonome central et périphérique. Dès lors, les produits capables d'intéragir avec les récepteurs adrénergiques peuvent induire un grand nombre de réponses physiologiques comme la vasoconstriction, la vasodilatation, l'augmentation ou la diminution du rythme cardiaque, la variation de la force de contraction du muscle cardiaque et des activités métaboliques. Différents composés adrénergiques ont été utilisés dans le passé pour modifier ces réponses physiologiques ou d'autres.
Dans le système nerveux central, la stimulation adrénergique est particulièrement utile pour compenser le déclin de la transmission neuronale induite dans le locus caeruleus par l'âge, la dépression ou les maladies dégénératives.

Les composés décrits dans la présente invention, possèdent un profil d'agonistes $\alpha_1$ adrénergiques. Cette propriété les rend utiles, comme l'ont indiqué P. TIMMERMANS et coll. dans "Comprehensive Medicinal Chemistry" (Vol. III, p. 134-185, 1990 - C. HANSH Editor, Pergamon, Oxford 1990) et J. NOZULAK et Coll. (J. Med. Chem., 35, 480-489, 1992), comme sympathomimétiques, dans le traitement de l'hypotension, de l'hypersomnie et des troubles de la vigilance et de la mémoire, ainsi que comme vasoconstricteurs et pour remédier aux symptômes apparaissant dans les maladies dégénératives telles que la maladie d'Alzheimer, la maladie de Parkinson et la chorée d'Huntington. L'utilité thérapeutique des produits de l'invention se base sur leur sélectivité pour les sous-types de récepteurs adrénergiques et leur modulation sélective des fonctions adrénergiques dans différents tissus et organes.

Plus spécifiquement la présente invention concerne les composés de formule (I) :

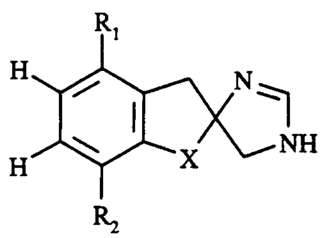

(I)

dans laquelle :

- X représente $-CH_2-$, $-(CH_2)_2-$, $-CH=CH-$, $-O-CH_2-$, $-S-CH_2-$, $-SO-CH_2-$ ou $-SO_2-CH_2-$,
- $R_1$ représente un atome d'hydrogène, d'halogène ou un groupement alkoxy ($C_1$-$C_6$) linéaire ou ramifié,
- $R_2$ représente un groupement alkoxy ($C_1$-$C_6$) linéaire ou ramifié ou alkylthio ($C_1$-$C_6$) linéaire ou ramifié,

leurs isomères ainsi que leurs sels d'addition à un acide pharmaceutiquement acceptable.

Parmi les acides pharmaceutiquement acceptables on peut citer à titre non limitatif les acides chlorhydrique, bromhydrique, sulfurique, phosphorique, acétique, lactique, malonique, succinique, fumarique, tartrique, maléique, citrique, méthane sulfonique, etc...

Parmi les isomères éventuels des composés de formule (I), on peut citer les énantiomères, les diastéréoisomères, les épimères ainsi que les tautomères.

L'invention s'étend également au procédé de préparation des composés de formule (I), caractérisé en ce que l'on utilise comme produit de départ un composé de formule (II) :

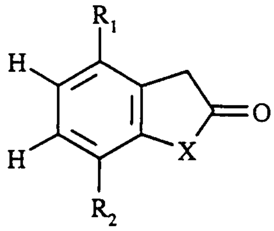

(II)

dans laquelle X, $R_1$ et $R_2$ ont la même signification que dans la formule (I) que l'on fait réagir :

- soit avec de la benzylamine en présence d'acide paratoluènesulfonique pour conduire au composé de formule (III) :

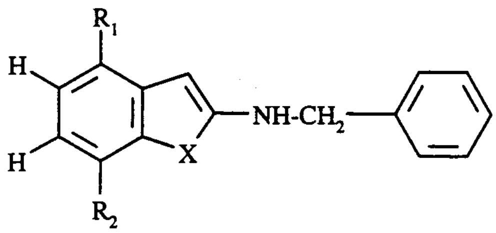

(III)

dans laquelle X, $R_1$ et $R_2$ ont la même signification que dans la formule (I) que l'on fait réagir, sous atmosphère inerte, avec du cyanure de triméthylsilyle en présence d'iodure de zinc, pour conduire au composé de formule (IV) :

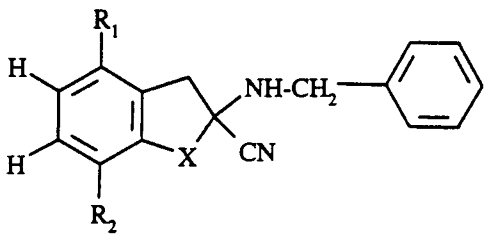

(IV)

dans laquelle X, $R_1$ et $R_2$ ont la même signification que dans la formule (I), que l'on réduit à l'aide d'hydrure de lithium aluminium, puis par hydrogénation catalytique pour conduire au composé de formule (V) :

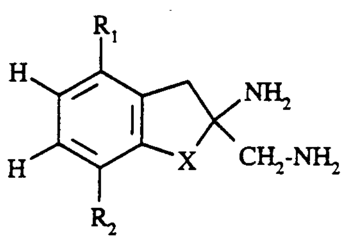

(V)

dans laquelle X, $R_1$ et $R_2$ ont la même signification que dans la formule (I),

- soit avec du cyanure de potassium en présence de chlorure d'ammonium en milieu inerte ou avec du cyanure de sodium en milieu acide, ou bien avec du cyanure de triméthylsilyle en présence d'iodure de zinc puis avec une solution alcoolique saturée en ammoniac,

pour conduire au composé de formule (VI) :

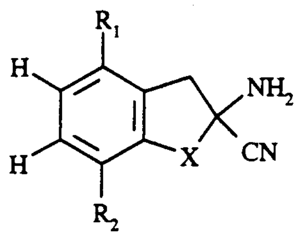

(VI)

dans laquelle X, $R_1$ et $R_2$ ont la même signification que dans la formule (I),
que l'on réduit à l'aide d'hydrure de lithium aluminium pour conduire au composé de formule (V) décrit précédemment,
composé de formule (V),
que l'on fait réagir avec de la formamidine en milieu alcoolique ou un formiate d'alkyle,
pour conduire au composé de formule (I),
que l'on purifie, le cas échéant, selon une technique classique de purification,
dont on sépare, si on le souhaite, les isomères selon une technique classique de purification,
et que l'on transforme, éventuellement, en leurs sels d'addition à un acide pharmaceutiquement acceptable.

La présente invention a également pour objet les compositions pharmaceutiques renfermant comme principe actif au moins un composé de formule générale (I) ou un de ses sels d'addition à un acide pharmacologiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiées, les comprimés sublingaux, les gélules, les tablettes, les suppositoires, les crèmes, pommades, gels dermiques, etc...

La posologie varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 0,1 et 1000 mg pour un traitement en 1 à 3 prises par 24 heures.

Les exemples suivants illustrent l'invention. Les produits de départ utilisés sont des produits connus ou préparés selon des modes opératoires connus.

Le produit décrit dans la préparation A conduit à un produit de départ utile dans la préparation d'un composé de l'invention.

**<u>Préparation A</u> : 5-Méthylthio-8-méthoxy-3,4-dihydro-naphtalèn-2(1H)-one**

**<u>Stade A</u> : Spiro[(1,3-dioxolane)-2 : 2'(8'-méthoxy-1',2',3',4'-tétrahydronaphtalène)]**

Une solution contenant 69 mmoles de 8-méthoxy-3,4-dihydro-naphtalèn-2(1H)-one, 75 mmoles d'éthylène glycol et 100 mg d'acide p.toluène sulfonique dans 500 ml de toluène est portée à reflux avec distillation azéotropique du mélange eau-toluène pendant 3 heures. Après refroidissement et évaporation sous vide, le résidu est repris par 100 ml d'éther éthylique. Après lavage à l'eau et évaporation, le produit attendu est obtenu sous forme d'huile par distillation du résidu sous vide.

<u>Point d'ébullition</u> : 122-125°C (p = 1,33 Pa)

**<u>Stade B</u> : Spiro[(1,3-dioxolane)-2 : 2'-(5'-iodo-8'-méthoxy-1',2',3',4'-tétrahydronaphtalène)]**

A une solution chauffée à 50°C contenant 35 mmoles du composé obtenu au stade précédent dans 200 ml d'acide acétique sont additionnées, goutte à goutte et simultanément, une solution de 108 mmoles d'iode dans 1 litre d'acide acétique et une solution de 56 mmoles de diacétate mercurique dans 1 litre d'acide acétique à une température ne dépassant pas 55°C. L'ensemble est agité 30 minutes à cette température, puis 2 heures à 20°C. L'acide acétique est évaporé sous vide et le résidu repris par une solution d'iodure de potassium à 10 %. Après filtration, le résidu est repris par une solution ammoniacale et extrait par du dichlorométhane. La phase organique est séchée, évaporée et conduit au produit attendu sous forme solide.

<u>Point de fusion</u> : 88-90°C

**Stade C : Spiro[(1,3-dioxolane)-2 : 2'-(5'-méthylthio-8'-méthoxy-1',2'3',4'-tétrahydronaphtalène)]**

A une suspension contenant 184 mmoles de méthanethiolate de lithium fraichement préparée dans 180 ml de diméthylsulfoxide (DMSO) sont ajoutées 18 mmoles du composé obtenu au stade précédent dans 90 ml de DMSO puis 286 mmoles d'oxyde cuivreux. Le mélange est agité 5 heures à 80°C, sous atmosphère d'azote. Après refroidissement, le précipité est filtré, le filtrat concentré et le résidu ainsi obtenu repris par du dichlorométhane. La phase organique est lavée à l'eau, séchée et évaporée. Le produit attendu est alors obtenu après purification du résidu par chromatographie sur colonne de silice en utilisant la dichlorométhane comme éluant.

Point de fusion : 42-44°C

**Stade D : 5-Méthylthio-8-méthoxy-3,4-dihydro-naphtalèn-2(1H)-one**

Une solution contenant 20 mmoles du composé obtenu au stade précédent dans 50 ml d'une solution aqueuse d'acide acétique à 30 % est chauffée 3 heures à 90°C. Après refroidissement, le mélange est versé sur de l'eau glacée. Le produit attendu est obtenu par filtration de précipité et lavage à l'eau.

Point de fusion : 88-90°C

**Exemple 1 : Spiro[(1,3-diazacyclopent-1-ène)-5 : 2'-(5',8'-diméthoxy-1',2',3',4'-tétrahydronaphtalène)], fumarate**

**Stade A : 2-Benzylamino-5,8-diméthoxy-3,4-dihydronaphtalène**

Un mélange contenant 82 mmoles de 5,8-diméthoxy-3,4-dihydro-naphtalèn-2(1H)-one, 82 mmoles de benzylamine et 0,20 g d'acide p.toluène sulfonique est porté au reflux avec distillation azéotropique du mélange eau/toluène. Après 2 heures de chauffage, le milieu est refroidi, filtré et le solvant évaporé sous vide et conduit au produit attendu sous forme d'huile.

**Stade B : 2-Benzylamino-2-cyano-5,8-diméthoxy-1,2,3,4-tétrahydronaphtalène**

A une solution maintenue sous azote contenant 80 mmoles du composé obtenu au stade précédent dans 400 ml de dichlorométhane sont ajoutées successivement 80 mmoles de cyanure de triméthylsilyle et 1 g d'iodure de zinc. Le mélange est agité à 20°C pendant une nuit puis lavé par de l'eau. Après séchage de la phase organique et évaporation, le produit attendu est obtenu sous forme d'huile.

**Stade C : 2-Benzylamino-2-aminométhyl-5,8-diméthoxy-1,2,3,4-tétrahydronaphtalène**

A une suspension contenant 140 mmoles d'hydrure de lithium aluminium dans 350 ml de tétrahydrofurane (THF) anhydre, 70 mmoles du composé obtenu au stade précédent dans 100 ml de THF anhydre sont ajoutées goutte à goutte à une température ne dépassant pas 20°C. Après 2 heures d'agitation, le mélange est refroidi à 0°C puis hydrolysé par addition successive de 6 ml d'eau, de 6 ml de soude 2N et de 12 ml d'eau. La suspension résultante est filtrée, le filtrat concentré. Le résidu huileux ainsi obtenu est dissous dans 200 ml d'acétate d'éthyle. Cette solution est lavée par de l'eau et extraite par une solution d'acide chlorhydrique 1N. La phase aqueuse est alcalinisée par de la soude à 35 % et extraite par de l'acétate d'éthyle. Après séchage et évaporation, on obtient le produit attendu sous forme d'huile.

**Stade D : 2-Amino-2-aminométhyl-5,8-diméthoxy-1,2,3,4-tétrahydronaphtalène**

Une suspension contenant 30 mmoles du composé obtenu au stade précédent, 125 mmoles de formiate d'ammonium et 6 g de palladium sur charbon à 10 % dans 250 ml de méthanol est chauffée au reflux sous agitation pendant 90 minutes. Après refroidissement, filtration du catalyseur et évaporation du solvant, on obtient le produit attendu sous forme d'huile.

**Stade E : Spiro[(1,3-diazacyclopent-1-ène)-5 : 2'-(5',8'-diméthoxy-1',2',3',4'-tétrahydronaphtalène)], fumarate**

Un mélange contenant 26 mmoles du composé obtenu au stade précédent, 27 mmoles d'acétate de formamidine dans 150 ml d'éthanol est agité à 20°C sous azote pendant 10 heures. Après évaporation du solvant, le résidu est repris par 100 ml d'acide chlorhydrique 1N. Cette phase organique est lavée par de l'acétate d'éthyle et alcalinisée par de la soude à 35 %. Après extraction par de l'acétate d'éthyle, les phases organiques sont lavées par une solution saturée de chlorure de sodium, concentrées et conduisent à un résidu solide. Ce résidu est dissous dans 80 ml d'éthanol et traité par un équivalent d'acide fumarique dissous dans 50 ml d'éthanol. Après évaporation du solvant, le produit

attendu cristallise dans de l'éthanol.

Point de fusion : 185-188°C

| Microanalyse élémentaire : | | | |
|---|---|---|---|
| | C % | H % | N % |
| calculé | 59,66 | 6,12 | 7,73 |
| trouvé | 59,41 | 6,07 | 7,74 |

Les exemples 2, 3 et 4 ont été obtenus selon le procédé décrit dans l'exemple 1 en utilisant les produits de départs correspondants.

**Exemple 2 : Spiro[(1,3-diazacyclopent-1-ène)-5 : 2'(5'-éthoxy-8'-méthoxy-1',2',3',4'-tétrahydronaphtalène)], fumarate**

Point de fusion : 174-178°C

| Microanalyse élémentaire : | | | |
|---|---|---|---|
| | C % | H % | N % |
| calculé | 58,06 | 6,03 | 6,45 |
| trouvé | 58,07 | 6,10 | 6,50 |

**Exemple 3 : Spiro[(1,3-diazacyclopent-1-ène)-5 : 2'-(5',8'-diéthoxy-1',2',3',4'-tétrahydronaphtalène)], fumarate**

Point de fusion : 196-198°C

| Microanalyse élémentaire : | | | |
|---|---|---|---|
| | C % | H % | N % |
| calculé | 61,53 | 6,71 | 7,17 |
| trouvé | 61,40 | 6,97 | 7,06 |

**Exemple 4 : Spiro[(1,3-diazacyclopent-1-ène)-5 : 2'-(4',7'-diméthoxyindane)], 1/2 fumarate**

Point de fusion : 224-228°C

| Microanalyse élémentaire : | | | |
|---|---|---|---|
| | C % | H % | N % |
| calculé | 62,06 | 6,25 | 9,65 |
| trouvé | 61,50 | 6,24 | 9,43 |

**Exemple 5 : Spiro[(1,3-diazacyclopent-1-ène)-5 : 2'-(5'-méthoxy-1',2',3',4'-tétrahydronaphtalène)], fumarate**

**Stade A : 2-Amino-2-cyano-5-méthoxy-1,2,3,4-tétrahydronaphtalène**

A une solution maintenue sous azote et sous agitation contenant 110 mmoles de 5-méthoxy-3,4-dihydro-naphtalèn-2(1H)-one dans 90 ml de méthanol et 50 ml d'eau, sont ajoutées successivement 112 mmoles de cyanure de potassium et 116 mmoles de chlorure d'ammonium. L'ensemble est agité 20 heures à 20°C puis concentré sous vide. Le résidu est repris par 200 ml d'acétate d'éthyle. Cette phase est alors lavée par de l'eau et extraite par de l'acide chlorhydrique 1N. La phase acide est alcalinisée par de la soude à 35 % et extraite par de l'acétate d'éthyle. Les phases organiques réunies sont séchées et évaporées sous vide et conduisent au produit attendu sous forme solide.

Point de fusion : 97°C

**Stade B : 2-Amino-2-aminométhyl-5-méthoxy-1,2,3,4-tétrahydronaphtalène**

A une suspension contenant 162 mmoles d'hydrure de lithium aluminium dans 200 ml de THF anhydre est additionnée, goutte à goutte, une solution contenant 70 mmoles du composé décrit au stade précédent dans 80 ml de THF, à une température ne dépassant pas 20°C. Après 1 heure d'agitation, le mélange refroidi à 0°C est hydrolysé par addition successive de 5,5 ml d'eau, 5,5 ml de soude 2N et 96 ml d'eau. La suspension résultante est filtrée et le filtrat évaporé. Le résidu huileux est alors dissous dans 250 ml d'acétate d'éthyle. Cette phase est lavée par de l'eau, extraite par de l'acide chlorhydrique 1N. Les phases aqueuses sont alors alcalinisées par de la soude à 35 % et extraites par de l'acétate d'éthyle. Après séchage et évaporation des phases organiques, le produit attendu est obtenu sous forme d'huile.

**Stade C : Spiro[(1,3-diazacyclopent-1-ène)-5 : 2'-(5'-méthoxy-1',2',3',4'-tétrahydronaphtalène)], fumarate**

Le produit attendu est obtenu selon le procédé décrit au stade E de l'exemple 1 à partir du composé décrit au stade précédent.

Point de fusion : 188-190°C

| Microanalyse élémentaire : | | | |
|---|---|---|---|
| | C % | H % | N % |
| calculé | 61,44 | 6,07 | 8,43 |
| trouvé | 61,23 | 6,02 | 8,34 |

**Exemple 6 : Spiro[(1,3-diazacyclopent-1-ène)-5 : 2'-(5'-méthylthio-8'-méthoxy-1',2',3',4'-tétrahydronaphtalène)], fumarate**

**Stade A : 2-Amino-2-cyano-5-méthylthio-8-méthoxy-1,2,3,4-tétrahydro naphtalène**

A un mélange, sous agitation, contenant 9 mmoles du composé décrit dans la préparation A et 29 mmoles de cyanure de sodium dans 50 ml d'eau et 40 ml d'éther éthylique est ajouté, goutte à goutte, 1 ml d'acide chlorhydrique concentré. Après 1 heure d'agitation à 20°C, la phase organique est décantée, lavée à l'eau, séchée et évaporée. L'huile obtenue est alors traitée par une solution méthanolique d'ammoniac (3,5 M), sous agitation, en milieu fermé, pendant 6 heures à 20°C. Après évaporation du solvant, l'huile est reprise par de l'éther éthylique et extraite par de l'acide chlorhydrique 1N. Les phases aqueuses sont alcalinisées par de la soude à 35 % puis extraites par de l'éther éthylique. Le produit attendu est alors obtenu sous forme solide après séchage et évaporation de la phase organique.

Point de fusion : 128-130°C

**Stade B : 2-Amino-2-aminométhyl-5-méthylthio-8-méthoxy-1,2,3,4-tétrahydro naphtalène**

Le produit attendu est obtenu selon le procédé décrit au stade B de l'exemple 5 en utilisant le composé décrit au stade précédent.

**Stade C : Spiro[(1,3-diazacyclopent-1-ène)-5 : 2'-(5'-méthylthio-8'-méthoxy-1',2',3',4'-tétrahydronaphtalène)], fumarate**

Le produit attendu est obtenu selon le procédé décrit au stade C de l'exemple 5 en utilisant le composé décrit au stade précédent.

Point de fusion : 188-190°C

| Microanalyse élémentaire : | | | | |
|---|---|---|---|---|
| | C % | H % | N % | S % |
| calculé | 57,13 | 5,86 | 7,40 | 8,47 |
| trouvé | 57,05 | 6,01 | 7,50 | 8,53 |

**Exemple 7 : Spiro [(1,3-diazacyclopent-1-ène)-5 : 2'-(5',8'-diméthoxy-1',2',3',4'-tétrahydronaphtalène)], fumarate, isomère α**

Le produit attendu est obtenu par dédoublement du composé décrit dans l'exemple 1 au moyen d'acide [+]-dibenzoyl-D-tartrique et par recristallisations successives dans l'éthanol. La pureté énantiomérique est contrôlée par chromatographie sur colonne chirale DIACEL-OG en utilisant comme éluant un mélange isopropanol/n-heptane/diéthylamine (20/80/0,1). Le sel est alors partagé entre la soude 1N et le dichlorométhane. La phase aqueuse est extraite par du dichlorométhane. Après séchage et évaporation des phases organiques, le résidu est repris par une solution éthanolique contenant un équivalent d'acide fumarique et l'ensemble est porté à reflux. Le produit attendu est obtenu après refroidissement et filtration sous forme d'un solide blanc.

Point de fusion : 187-188°C

| Microanalyse élémentaire : | | | |
|---|---|---|---|
| | C % | H % | N % |
| calculé | 59,66 | 6,12 | 7,73 |
| trouvé | 59,59 | 6,08 | 7,65 |

**Exemple 8 : Spiro [(1,3-diazacyclopent-1-ène)-5 : 2'-(5',8'-diméthoxy-1',2',3',4'-tétrahydronaphtalène)], fumarate, isomère β**

Le produit attendu est obtenu selon le procédé décrit dans l'exemple 7 par dédoublement du composé décrit dans l'exemple 1 au moyen d'acide [-]-dibenzoyl-L-tartrique.

Point de fusion : 187-188°C

| Microanalyse élémentaire : | | | |
|---|---|---|---|
| | C % | H % | N % |
| calculé | 59,66 | 6,12 | 7,73 |
| trouvé | 59,36 | 6,09 | 7,74 |

**Etude pharmacologique des composés de l'invention**

**Exemple 9 : Etude in vitro sur les artères fémorales et les veines saphènes de chien**

La technique utilisée s'inspire de celle décrite par FOWLER et Coll. (J. Pharmacol. Exp. Ther., 229, 712-718,

1984). Des chiens bâtards mâles ou femelles d'environ 15-25 kg ont été utilisés comme source d'organes. Les animaux sont anesthésiés au pentobarbital (30 mg/kg, en intraveineux). Les pates sont incisées et les vaisseaux prélevés. Ils sont placés dans du liquide de Krebs-Ringer (NaCl 118 mM ; $NaHCO_3$ 25 mM ; Glucose 10 mM ; KCl 4,7 mM; $CaCl_2$ 1,25 mM ; $MgSO_4$ 1,19 mM ; $KH_2PO_4$ 1,14 mM) à température ambiante et sous bullage de carbogène (95 % $0_2$, 5 % $CO_2$). Ces vaisseaux sont alors soigneusement débarrassés de leur graisse puis découpés en anneaux de 2 mm de large et montés sous une tension de base de 4 g (artères fémorales) ou 1 g (veines saphènes) dans des cuves thermostatées à 37°C contenant du liquide de Krebs-Ringer constamment bullé par du carbogène. Un crochet inférieur constitue le point fixe, tandis que le crochet supérieur est relié à un capteur de force isométrique. Les variations de tension sont digitalisées, stockées sur disque et traitées par un système informatique. Après montage, les organes sont laissés en repos pendant 90 minutes, des rinçages étant effectués toutes les 30 min. Après réajustement de la tension de base, une contraction est provoquée par une dose unique de KCl (100 mM). Après stabilisation, lavages et retour à la ligne de base, une contraction est provoquée par une dose unique de phényléphrine (concentration submaximale) afin de régulariser les contractions suivantes. Après lavage et retour à la ligne de base, une courbe effet/concentration est réalisée par une adjonction de doses cumulatives d'agoniste (l'espacement entre les doses est d'un demi-log). Cette expérience permet de calculer la concentration efficace 50 % ($EC_{50}$) de la manière suivante : les valeurs de tension sont d'abord converties en pourcentages par rapport à l'effet maximum provoqué par 100 mM de KCl. Cette $EC_{50}$ est déterminée par régression non linéaire par la méthode SIMPLEX (M.S. CACECI, Byte, 340-362, 1984), calculée suivant le modèle de la loi d'action de masse de L. MICHAELIS et M.L. MENTEN (Biochem. Zeitschrifft, 49, 333-369, 1913).

$$E = (Emax*C^{n})(EC^{n}+C^{n})$$ avec E = effet ; Emax = effet maximum ;
C = concentration ; EC = $EC_{50}$ ; n = nombre de HILL

Les produits de l'invention contractent les artères et les veines de chien. Le maximum de ces contractions se rapproche de celui obtenu avec le KCl. Les résultats obtenus sont présentés dans le tableau ci-dessous :

| | ARTERE | | VEINE | |
|---|---|---|---|---|
| Exemple | $EC_{50}$ (µM) | Max (% KCl) | $EC_{50}$ (µM) | Max (% KCl) |
| 1 | 0,16 | 72 | 0,2 | 100 |
| 5 | 1,5 | 93 | 6,4 | 100 |
| 6 | 0,08 | 85 | 0,8 | 98 |
| 7 | 0,03 | 95 | 0,12 | 109 |

**<u>Exemple 10</u> : Etude in vivo chez le RAT AMYELE**

Des rats Sprague Dawley mâles (300-400 g) sont anesthésiés à l'éther. La trachée est canulée, la moëlle épinière est détruite au moyen d'une tige en acier et l'animal est immédiatement mis sous respiration artificielle. Les nerfs vagues sont sectionnés. Les artères carotides sont ligaturées, un cathéter est placé dans l'une et sert à enregistrer la pression artérielle. Trois autres cathéters sont placés dans les veines jugulaires et la veine du pénis et servent aux injections. La température des animaux est maintenue à 36°C. L'animal est prétraité par une injection de tertatolol (100 µg/kg). L'animal est également prétraité 10 minutes après par le prazosin (100 µg/kg) ou la yohimbine (1 mg/kg) lorsque l'on veut déterminer les propriétés $alpha_1$ ou $alpha_2$-adrénergiques du produit. Dix minutes plus tard, des doses cumulatives croissantes de produit sont injectées toutes les 20 secondes. Les variations de pression artérielles sont détectées à l'aide d'une cellule de pression P23XL Statham et sont enregistrées. Les valeurs de pression sont exprimées en mm Hg. Cette expérience permet de calculer la concentration augmentant la pression de 20 mm Hg ($C_{20}$) par régression non linéaire suivant le modèle de la loi d'action de masse de Michaelis et Menten comme décrit ci-dessus. L'effet maximum obtenu est ensuite converti en pourcentage par rapport à l'effet maximum provoqué par la phényléphrine. Les composantes $alpha_1$ ou $alpha_2$-adrénergiques du produit sont appréciées à l'aide du rapport des $C_{20}$ obtenues en présence de prazosin ou de yohimbine sur les valeurs obtenues en absence de ces antagonistes. Chez le rat amyélé, les produits de l'invention induisent des hypertensions qui sont inhibées plus par le prazosin que par la yohimbine. Les résultats sont rassemblés dans le tableau ci-dessous :

| | $C_{20}$ (µg/kg) | Ratio $C_{20}$ traité / $C_{20}$ contrôle | |
|---|---|---|---|
| Exemple | Contrôle | Prazosin | Yohimbine |
| 1 | 0,009 | 97 | 11 |
| 2 | 0,06 | 22 | 5 |
| 3 | 0,6 | 24 | 8 |

**<u>Exemple 11</u> : Composition pharmaceutique**

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I) :

(I) :

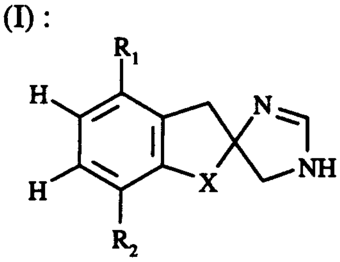

(I)

dans laquelle :

- X représente $-CH_2-$, $-(CH_2)_2-$, $-CH=CH-$, $-O-CH_2-$, $-S-CH_2-$, $-SO-CH_2-$ ou $-SO_2-CH_2-$,
- $R_1$ représente un atome d'hydrogène, d'halogène ou un groupement alkoxy ($C_1$-$C_6$) linéaire ou ramifié,
- $R_2$ représente un groupement alkoxy ($C_1$-$C_6$) linéaire ou ramifié ou alkylthio ($C_1$-$C_6$)

linéaire ou ramifié, leurs isomères ainsi que leurs sels d'addition à un acide pharmaceutiquement acceptable.

**2.** Composés de formule (I) selon la revendication 1 tels que X représente $-(CH_2)_2-$, leurs isomères ainsi que leurs sels d'addition à un acide pharmaceutiquement acceptable.

**3.** Composés de formule (I) selon la revendication 1 qui est le spiro[(1,3-diazacyclopent-1-ène)-5 : 2'-(5',8'-diméthoxy-1',2',3',4'-tétrahydronaphtalène)], ses isomères ainsi que ses sels d'addition à un acide pharmaceutiquement acceptable.

**4.** Procédé de préparation des composés de formule (I) selon la revendication 1, caractérisé en ce que l'on utilise

comme produit de départ un composé de formule (II) :

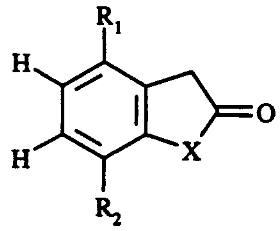

(II)

dans laquelle X, $R_1$ et $R_2$ ont la même signification que dans la formule (I) que l'on fait réagir :

- soit avec de la benzylamine en présence d'acide paratoluènesulfonique pour conduire au composé de formule (III) :

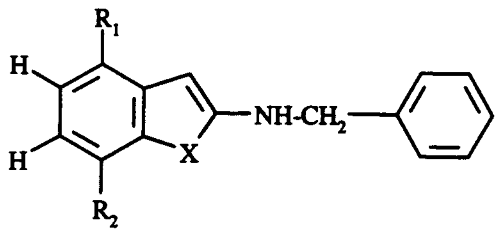

(III)

dans laquelle X, $R_1$ et $R_2$ ont la même signification que dans la formule (I) que l'on fait réagir, sous atmosphère inerte, avec du cyanure de triméthylsilyle en présence d'iodure de zinc, pour conduire au composé de formule (IV) :

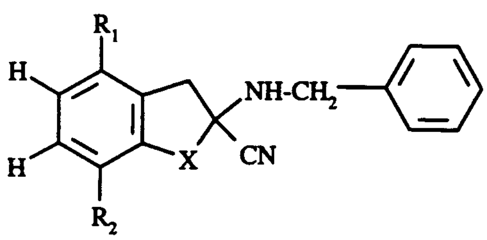

(IV)

dans laquelle X, $R_1$ et $R_2$ ont la même signification que dans la formule (I), que l'on réduit à l'aide d'hydrure de lithium aluminium, puis par hydrogénation catalytique pour conduire au composé de formule (V) :

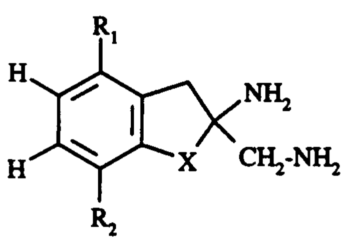

(V)

dans laquelle X, $R_1$ et $R_2$ ont la même signification que dans la formule (I),

- soit avec du cyanure de potassium en présence de chlorure d'ammonium en milieu inerte ou avec du cyanure

de sodium en milieu acide, ou bien avec du cyanure de triméthylsilyle en présence d'iodure de zinc puis avec une solution alcoolique saturée en ammoniac,
pour conduire au composé de formule (VI) :

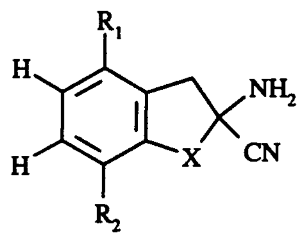

(VI)

dans laquelle X, $R_1$ et $R_2$ ont la même signification que dans la formule (I),
que l'on réduit à l'aide d'hydrure de lithium aluminium pour conduire au composé de formule (V) décrit précédemment,
composé de formule (V),
que l'on fait réagir avec de la formamidine en milieu alcoolique ou un formiate d'alkyle,
pour conduire au composé de formule (I),
que l'on purifie, le cas échéant, selon une technique classique de purification,
dont on sépare, si on le souhaite, les isomères selon une technique classique de purification, et que l'on transforme, éventuellement, en leurs sels d'addition à un acide pharmaceutiquement acceptable.

5. Compositions pharmaceutiques contenant comme principe actif au moins un composé selon l'une quelconque des revendications 1 à 3 seul ou en combinaison avec un ou plusieurs excipients ou véhicules inertes, non toxiques, pharmaceutiquement acceptables.

6. Compositions pharmaceutiques selon la revendication 5 contenant au moins un principe actif selon l'une quelconque des revendications 1 à 3 utilisé en tant qu'agoniste $\alpha_1$ adrénergique dans le traitement de l'hypotension, de l'hypersomnie, des troubles de la vigilance et de la mémoire ainsi que des symptômes apparaissant dans les maladies dégénératives.

## Claims

1. Compounds of formula (I):

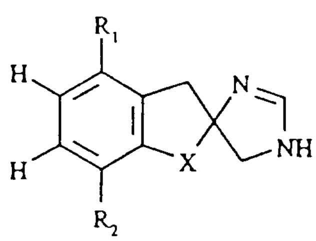

(I)

wherein:

- X represents $-CH_2-$, $-(CH_2)_2-$, $-CH=CH-$, $-O-CH_2-$, $-S-CH_2-$, $-SO-CH_2-$ or $-SO_2-CH_2-$,
- $R_1$ represents a hydrogen or halogen atom or a linear or branched $(C_1-C_6)$alkoxy group,
- $R_2$ represents a linear or branched $(C_1-C_6)$alkoxy or linear or branched $(C_1-C_6)$alkylthio group,

their isomers, and also addition salts thereof with a pharmaceutically acceptable acid.

2. Compounds of formula (I) according to claim 1 wherein X represents $-(CH_2)_2-$, their isomers, and also addition salts thereof with a pharmaceutically acceptable acid.

3. Compound of formula (I) according to claim 1 which is spiro[(1,3-diazacyclopent-1-ene-5 : 2'-(5',8'-dimethoxy-1',2',3',4'-tetrahydronaphthalene)], its isomers, and also addition salts thereof with a pharmaceutically acceptable acid.

**4.** Process for the preparation of compounds of formula (I) according to claim 1, characterised in that there is used as starting material a compound of formula (II):

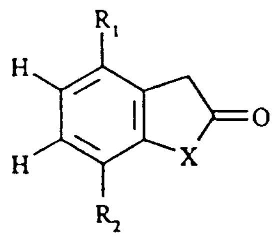

(II)

wherein X, $R_1$ and $R_2$ are as defined for formula (I)
which is reacted <u>either</u> with:

- benzylamine in the presence of *para*-toluenesulphonic acid to yield a compound of formula (III):

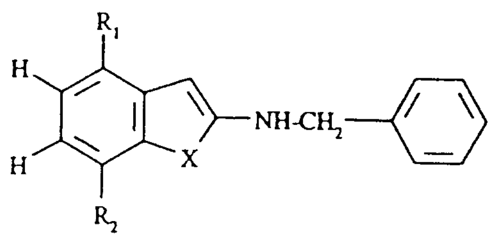

(III)

wherein X, $R_1$ and $R_2$ are as defined for formula (I)
which is reacted, under an inert atmosphere, with trimethylsilyl cyanide in the presence of zinc iodide, to yield a compound of formula (IV):

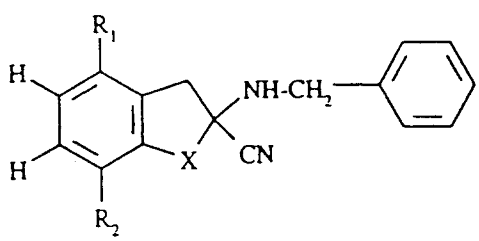

(IV)

wherein X, $R_1$ and $R_2$ are as defined for formula (I),
which is reduced using lithium aluminum hydride, then by catalytic hydrogenation to yield a compound of formula (V):

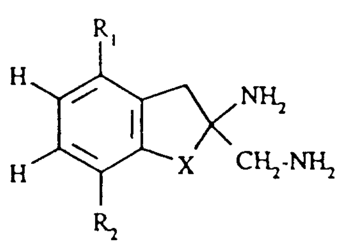

(V)

wherein X, $R_1$ and $R_2$ are as defined for formula (I),

or with

- potassium cyanide in the presence of ammonium chloride in an inert medium or with sodium cyanide in an acid medium, or with trimethylsilyl cyanide in the presence of zinc iodide and then with an alcoholic solution saturated by ammonia, to yield a compound of formula (VI):

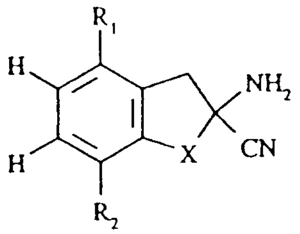

(VI)

wherein X, $R_1$ and $R_2$ are as defined for formula (I),
which is reduced using lithium aluminium hydride to yield a compound of formula (V) defined above,
which compound of formula (V) is reacted with formamidine in an alcoholic medium or with an alkyl formate, to yield a compound of formula (I)
which is purified, where appropriate, in accordance with a conventional purification method,
separated, if desired, into its isomers in accordance with a conventional purification method,
and transformed, optionally, into addition salts thereof with a pharmaceutically acceptable acid.

**5.** Pharmaceutical compositions comprising as active ingredient at least one compound according to any one of claims 1 to 3 on its own or in combination with one or more pharmaceutically acceptable inert non-toxic excipients or carriers.

**6.** Pharmaceutical compositions according to claim 5 comprising at least one active ingredient according to any one of claims 1 to 3 for use as an $\alpha_1$-adrenergic agonist in the treatment of hypotension, hypersomnia, attention and memory disorders, and also of symptoms occurring in degenerative diseases.

**Patentansprüche**

**1.** Verbindungen der Formel (I):

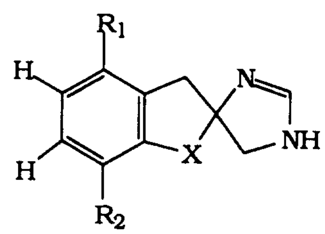

(I)

in der:

- X $-CH_2-$, $-(CH_2)_2-$, $-CH=CH-$, $-O-CH_2-$, $-S-CH_2-$, $-SO-CH_2-$ oder $-SO_2-CH_2-$,
- $R_1$ ein Wasserstoffatom, ein Halogenatom oder eine geradkettige oder verzweigte ($C_1$-$C_6$)-Alkoxygruppe und
- $R_2$ eine geradkettige oder verzweigte ($C_1$-$C_6$)-Alkoxygruppe oder eine geradkettige oder verzweigte ($C_1$-$C_6$)-Alkylthiogruppe bedeuten,

deren Isomere sowie deren Additionssalze mit einer pharmazeutisch annehmbaren Säure.

**2.** Verbindungen der Formel (I) nach Anspruch 1, in der X-$(CH_2)_2$- bedeutet, deren Isomere sowie deren Additionssalze mit einer pharmazeutisch annehmbaren Säure.

**3.** Verbindungen der Formel (I) nach Anspruch 1, nämlich Spiro[(1,3-diazacyclopent-1-en)-5 : 2'-(5',8'-dimethoxy-1',2',3',4'-tetrahydronaphthalin)], dessen Isomere und dessen Additionssalze mit einer pharmazeutisch annehmbaren Säure.

4. Verfahren zur Herstellung der Verbindungen der Formel (I) nach Anspruch 1, dadurch gekennzeichnet, daß man als Ausgangsprodukt eine Verbindung der Formel (II) verwendet:

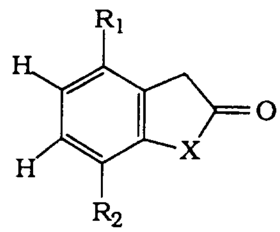

(II)

in der X, $R_1$ und $R_2$ die bezüglich der Formel (I) angegebenen Bedeutungen besitzen,
welche man:

- <u>entweder</u> mit Benzylamin in Gegenwart von p-Toluolsulfonsäure umsetzt zur Bildung der Verbindung der Formel (III):

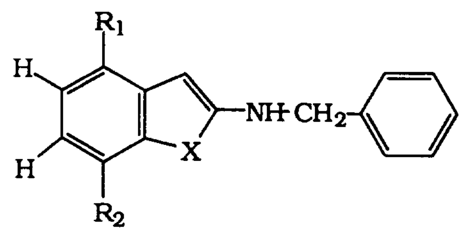

(III)

in der X, $R_1$ und $R_2$ die bezüglich der Formel (I) angegebenen Bedeutungen besitzen,
welche man unter einer inerten Atmosphäre mit Trimethylsilylcyanid in Gegenwart von Zinkiodid umsetzt zur Bildung der Verbindung der Formel (IV):

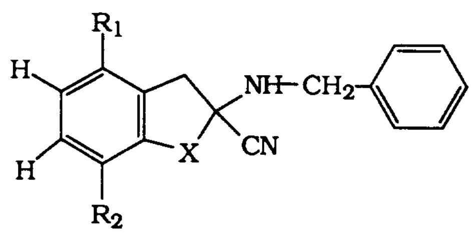

(IV)

in der X, $R_1$ und $R_2$ die bezuglich der Formel (I) angegebenen Bedeutungen besitzen, welche man mit Lithiumaluminiumhydrid und dann durch katalytische Hydrierung reduziert zur Bildung der Verbindung der Formel (V):

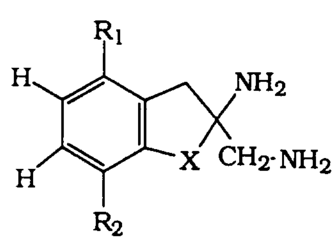

(V)

in der X, $R_1$ und $R_2$ die bezüglich der Formel (I) angegebenen Bedeutungen besitzen,

- <u>oder</u> mit Kaliumcyanid in Gegenwart von Ammoniumchlorid in einem inerten Medium oder mit Natriumcyanid

in saurem Medium oder mit Trimethylsilylcyanid in Gegenwart von Zinkiodid und dann mit einer gesättigten alkoholischen Ammoniaklösung umsetzt zur Bildung der Verbindung der Formel (VI):

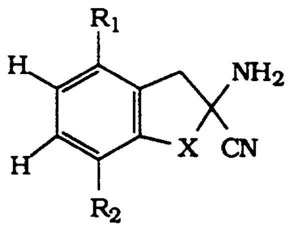

(VI)

in der X, $R_1$ und $R_2$ die bezüglich der Formel (I) angegebenen Bedeutungen besitzen,
welche man mit Lithiumaluminiumhydrid reduziert zur Bildung der oben beschriebenen Verbindung der Formel (V),
welche Verbindung der Formel (V) man
mit Formamidin in alkoholischem Medium oder mit einem Alkylformiat umsetzt zur Bildung der Verbindung der Formel (I),
welche man gegebenenfalls mit Hilfe einer klassischen Reinigungsmethode reinigt,
welche man gewünschtenfalls mit Hilfe einer klassischen Reinigungsmethode in die Isomeren aufspaltet und welche man gegebenenfalls mit Hilfe einer pharmazeutisch annehmbaren Säure in ihre Additionssalze überführt.

5. Pharmazeutische Zubereitungen enthaltend als Wirkstoff mindestens eine Verbindung nach irgendeinem der Ansprüche 1 bis 3 allein oder in Kombination mit einem oder mehreren inerten, nichttoxischen, pharmazeutisch annehmbaren Trägermaterialien oder Bindemitteln.

6. Pharmazeutische Zubereitungen nach Anspruch 5 enthaltend mindestens einen Wirkstoff nach einem der Ansprüche 1 bis 3 zur Verwendung als $\alpha_1$-adrenergischen Agonisten bei der Behandlung der Hypotension, der Hypersomnie, von Störungen des Verhaltens und des Gedächtnisses sowie von Symptomen, die bei degenerativen Erkrankungen auftreten.